# EUROPÄISCHE PATENTSCHRIFT

(11) **EP 0 402 727 B1**
(45) Veröffentlichungstag und Bekanntmachung des Hinweises auf die Patenterteilung: **03.03.1993**
(21) Anmeldenummer: 90110543.7
(22) Anmeldetag: 02.06.1990
(51) Int. Cl.: B01J 37/18, B01J 23/74, C07C 209/48, B01J 23/89, B01J 23/76

(54) **Verfahren zur Herstellung eines Eisenkatalysators und ein Verfahren zur Herstellung von primären Aminen durch Hydrierung von Nitrilen unter Verwendung dieses Eisenkatalysators**
Process to prepare an iron catalyst and process to prepare primary amines by hydrogenation of nitriles using said iron catalyst
Procédé pour préparer un catalyseur à base de fer et procédé de préparation d'amines primaires pour hydrogénation de nitriles en utilisant ce catalyseur au fer

(30) Priorität: 16.06.1989 DE 3919694
(43) Veröffentlichungstag der Anmeldung: 19.12.1990
(73) Patentinhaber: BAYER AG, 51368 Leverkusen (DE)
(72) Erfinder: Immel, Otto, Dr., D-4150 Krefeld (DE); Liebsch, Dietrich, Dr., D-5090 Leverkusen (DE); Schwarz, Hans-H., Dr., D-4150 Krefeld (DE); Wendel, Stephan, Dr., D-5090 Leverkusen (DE); Fischer, Peter, Dr., D-5068 Odenthal-Osenau (DE)

(56) Entgegenhaltungen:
- EP-A- 0 151 291
- DE-A- 1 085 165
- DE-A- 2 034 380
- GB-A- 2 092 016
- US-A- 4 301 032
- US-A- 4 480 051

## Beschreibung

Die Erfindung betrifft ein neues Verfahren zur Herstellung eines geformten Eisenkatalysators durch oberflächliche Anoxidation von Eisen oder Eisenlegierungen in Form von Spänen, Granulat oder anderer stückiger Form und anschließende Reduktion durch Hydrierung, sowie ein Verfahren zur Herstellung von primären Aminen, insbesondere von Hexamethylendiamin durch katalytische Hydrierung der entsprechenden Nitrile unter Verwendung von derart hergestellten Eisenkatalysatoren.

Die Verwendung von Eisenkatalysatoren für die Hydrierung von Nitrilen, insbesondere von Adiponitril, zu den entsprechenden Aminen ist seit langem bekannt. So wird in DE-PS 848 654 ein Hydrierverfahren für Adiponitril beschrieben, bei dem ein Eisenkatalysator verwendet wurde, der auf Bimsstein niedergeschlagen und bei erhöhter Temperatur reduziert wurde. In der DE-OS 2 429 293 wird zur Herstellung eines Katalysators ein schwedisches Magnetiterz bei 1590°C geschmolzen. Nach dem Erstarren der Eisenoxidschmelze wird die erstarrte Masse zerkleinert, das Granulat mit Wasserstoff reduziert und als Katalysator für die Hydrierung von Adiponitril verwendet. Auch nach dem in der DE-AS 2 034 380 beschriebenen Verfahren zur Hydrierung von Adiponitril wird ein Eisenkatalysator verwendet, der durch Reduktion von stückigem Eisenoxid, z.B. von Eisenerz, das in der Natur in Labrador vorkommt, hergestellt wurde.

In der EP-A-0 101 584 wird für die Hydrierung von Adiponitril eine geformte Eisenkatalysatormasse vorgeschlagen, die metallische Eisenteilchen enthält, die aus anisometrischen Eisenoxidteilchen durch Reduktion mit Wasserstoff erhalten worden sind, Es handelt sich dabei um ein relativ aufwendiges Verfahren zur Herstellung eines Eisenkatalysators. Zudem führt der Zerfall der zu Formkörpern verpreßten Eisenteilchen zu einem störenden Druckanstieg bei einem kontinuierlichen Hydrierverfahren.

Es war daher die der Erfindung zugrundeliegende Aufgabe ein einfaches Verfahren zur Hydrierung von Nitrilen, d.h. insbesondere, einen Katalysator für ein solches Verfahren zur Verfügung zu stellen, der einfach herstellbar ist und die Vorteile einer hohen Aktivität und einer langen Lebensdauer in sich vereint, Weiterhin sollte die schwierige Phase einer Formgebung des Katalysators vermieden werden durch Verwendung von metallischem Eisen in form von für heterogen katalytische Prozesse geeigneten Partikeln. Diese Aufgabe konnte durch das nachstehend beschriebene Verfahren zur Herstellung eines Eisenkatalysators bzw. von primären Aminen durch Hydrierung der entsprechenden Nitrile gelöst werden.

Gegenstand der Erfindung ist ein Verfahren zur Herstellung eines geformten Eisenkatalysators zur Hydrierung von Nitrilen, dadurch gekennzeichnet, daß man Eisenschwamm, der mindestens zu 85% aus Eisen bestet und bei der Direktreduktion von Eisenerz unterhalb der Schmelztemperatur anfällt, in Form von Granulat oder einer anderen stückigen Form mittels gasförmigen Sauerstoff bei 200 bis 800° C bis zu einer Gewichtszunahme von mindestens 5 % und maximal 32 % anoxidiert und anschließend im Wasserstoffstrom, der gegebenenfalls untergeordnete Mengen an nicht oxidierenden Gasen enthalten kann, bei 200 - 500° C reduziert.

Gegenstand der Erfindung ist auch ein Verfahren zur Herstellung von primären Aminen durch katalytische Hydrierung der entsprechenden Nitrile, dadurch gekennzeichnet, daß man als Hydrierkatalysatoren nach diesem Verfahren erhältliche Eisenkatalysatoren verwendet.

Es war zwar aus der US-PS 4 301 032 bereits bekannt, Hydrierkatalysatoren durch Oxidation und anschließende Hydrierung von Metallen herzustellen, wobei als Ausgangslegierungen thoriumhaltige Legierungen mit Elementen der achten Nebengruppe des Periodensystems zum Einsatz gelangen. Gemäß Ausführungsbeispielen handelt es sich bei den einzusetzenden Metallegierungen insbesondere um solche aus Thorium und Nickel.

Irgendwelche Hinweise auf das erfindungsgemäße Verfahren zur Herstellung von Hydrierkatalysatoren können somit diesem Dokument nicht entnommen werden.

Gemäß US-PS 4 480 051 werden zur Herstellung von Hydrierkatalysatoren Eisenoxide bei höheren Temperaturen abwechselnd hydriert und oxidiert, wobei die hier zum Einsatz gelangenden Reaktionstemperaturen 600° C nicht übersteigen dürfen. Wie eigene Versuche des Anmelders ergaben, führt die Verwendung von derart hergestellten Eisenkatalysatoren bei der Hydrierung von Adipinsäuredinitril zu vergleichsweise niedrigen Ausbeuten unter Bildung beträchtlicher Mengen an unerwünschten Nebenprodukten.

Als Ausgangsmaterial zur Herstellung der erfindungsgemäßen Eisenkatalysatoren kommt Eisenschwamm zum Einsatz, der bei der sogenannten Direktreduktion von Eisenerz unterhalb der Schmelztemperatur der Rohstoffe anfällt.

Das Eisen wird beim Verfahren zur Herstellung des Eisenkatalysators in Form von Granulat, Spänen oder in einer anderen geeigneten stückigen Form eingesetzt.

Dieses Eisen wird, gegebenenfalls nach erfolgter Dotierung mit einem als Aktivator wirkenden Metall in Gegenwart von gasförmigem Sauerstoff, vorzugsweise in Gegenwart von Luft, bei 200 bis 800°C, vorzugsweise 400 bis 800°C getempert bis es eine Gewichtszunahme von mindestens 5 und maximal 32 %, vorzugsweise von 10 bis 30 % erfährt. Im allgemeinen ist dies während eines Zeitraums von 5 bis 100 Stunden der Fall, Die Dauer der Temperung hängt selbstverständlich von der Temperatur, der Sauerstoffkonzentration und der chemischen Zusammensetzung des Eisens ab.

Das auf diese Weise anoxidierte Eisen wird in einem zweiten Reaktionsschritt im Wasserstoffstrom, der auch andere, nicht oxidierende Gase wie z.B. Ammoniak, Methan oder Kohlenoxid in untergeordneten Mengen enthalten kann, bei erhöhter Temperatur reduziert. Diese Reduktion erfolgt im allgemeinen bei einer Temperatur von 200 bis 500°C, vorzugsweise 250 bis 450°C unter einem Wasserstoffdruck von bis zu 400, vorzugsweise 1 bis 350 bar. Vorzugsweise erfolgt die Reduktion so lange, bis eine größere Menge des im ersten Reaktionsschritt gebildeten Eisenoxids zu elementarem Eisen reduziert ist. Dies ist im allgemeinen während eines Zeitraums von 3 bis 100 Stunden der Fall. Eine nur teilweise Reduktion des in der ersten Reaktionsstufe gebildeten Eisenoxids würde jedoch ebenfalls zu einem brauchbaren Katalysator führen, so daß eine vollständige Reduktion des vorliegenden Eisenoxids nicht zwingend erforderlich ist.

Gemäß einer besonderen Ausführungsform des erfindungsgemäßen Verfahrens zur Herstellung des Eisenkatalysators erfolgt die Reduktion des oxidierten Eisens im gleichen Reaktionsraum, in dem anschließend die Hydrierung des Nitrils erfolgt. Die geometrische Form des hergestellten Hydrierkatalysators entspricht in Form und Gestalt weitgehend dem eingesetzten Ausgangsmaterial. Bei der Wahl der Form des Ausgangsmaterials kann man sich nach den strömungstechnischen Gegebenheiten richten, die man im Hydrierreaktor erwartet. Bei dieser Verfahrensweise erspart man sich das Einfüllen des luftempfindlichen Katalysators in den Hydrierreaktor.

Gemäß einer weiteren bevorzugten Ausführungsform des erfindungsgemäßen Verfahrens zur Herstellung des Eisenkatalysators wird das Eisen vor oder nach der Temperung, jedoch vor der Hydrierung, mit als Aktivator wirkenden Metallen dotiert. Derartige Metalle sind beispielsweise Kobalt, Mangan, Chrom, Molybdän, Ruthenium oder Iridium.

Die Dotierung erfolgt dergestalt, daß man das Eisen vor oder im Anschluß an die Temperung mit einer wäßrigen Lösung eines Salzes, beispielsweise eines Nitrats, Acetats oder Formiats eines derartigen Metalls, tränkt oder befeuchtet und anschließend an der Luft, gegebenenfalls bei erhöhter Temperatur von beispielsweise 60 bis 120°C trocknet. Die Menge der beispielhaft genannten Salze entspricht dabei im allgemeinen 0,01 bis 1 Gew.-% aktivierendes Metall, bezogen auf unoxidiertes Eisen bzw. unoxidierte Eisenlegierung.

Im Anschluß an die Dotierung erfolgt dann der jeweils nächste Schritt, d.h. die Temperung oder, falls die Dotierung nach der Temperung erfolgte, die Hydrierung.

Die erfindungsgemäß zugänglichen Eisenkatalysatoren eignen sich insbesondere hervorragend als Katalysatoren für die Hydrierung von Nitrilen zu den entsprechenden Aminen. Von technischer Bedeutung ist hier insbesondere die Hydrierung von Adiponitril zu Hexamethylendiamin. Auch andere Nitrile können mit dem erfindungsgemäßen Katalysator hydriert werden, z.B. Acetonitril, Propionsäurenitril, Bernsteinsäuredinitril, Glutarsäuredinitril, Benzoesäurenitril oder Nicotinsäurenitril. Um die Bildung unerwünschter Nebenprodukte zu vermeiden, wird die Hydrierung im allgemeinen in Gegenwart von Ammoniak durchgeführt. Dabei wird das Nitril, vorzugsweise das Adiponitril und Ammoniak im Gewichtsverhältnis 1:0,1 bis 1:10, vorzugsweise 1:0,5 bis 1:6, eingesetzt. Die Hydrierung erfolgt im allgemeinen bei einer Temperatur von 80 bis 180°C unter einem Druck von 100 bis 450 bar. Die Hydrierung kann in An- oder Abwesenheit von geeigneten Lösungsmitteln wie Methanol, Ethanol oder Butanol in Autoklaven oder in kontinuierlich betriebenen Druckreaktoren erfolgen. Die Aufarbeitung der bei der Hydrierung im allgemeinen in Ausbeuten von über 90 % anfallenden primären Amine, insbesondere des Hexamethylendiamins, erfolgt in an sich bekannter Weise destillativ.

In den nachfolgenden Beispielen beziehen sich alle Prozentangaben auf Gewichtsprozente.

### Beispiele

### Beispiel 1

Ein handelsübliches, nach dem Midrex-Verfahren (vergl. z.B. Ullmanns Enzyklopädie der technischen Chemie, 4. Auflage, Band 10, Seiten 396 ff.) hergestelltes Eisengranulat folgender Zusammensetzung:
- Fe:: 90 - 92%
- C:: 1,5 - 2,5 %
- SiO₂:: 2,2 %
- Al₂O₃:: 1,0 %
- P:: 0,03 %
- CaO:: 1,1 %
- MgO:: 0,8 %
- MnO:: 0,25 %
- TiO₂:: 0,2 %

wurde durch Mahlen und Sieben auf eine Korngröße von 1 bis 5 mm gebracht und 22 Stunden im Glühofen bei einer Ofentemperatur von 620°C unter Luftzutritt getempert (Gewichtszunahme: 27,7 %).

30 g des getemperten Eisens wurde in einem Glasrohr 3 Stunden mit einem großen Überschuß an Wasserstoff (20 l/h) bei 380°C reduziert. Der so hergestellte Katalysator wurde im Wasserstoffstrom abgekühlt und unter Luftausschluß in einen 0,25 l-Schüttelautoklaven gegeben. Weiterhin wurde der Autoklav mit 40 g Adiponitril und 40 g flüssigem Ammoniak beschickt. Es wurde Wasserstoff aufgedrückt und die Hydrierung bei 140°C und 230 bis 300 bar durchgeführt. Die Hydrierung wurde nach 4 Stunden beendet. Es entstand ein Hydrierprodukt folgender Zusammensetzung:
- Hexamethylendiamin: 97,9 %
- Bishexamethylentriamin: 0,3 %
- Azacycloheptan: 0,8 %
- 1,2-Diaminocyclohexan: 0,1 %
- unbekannte Verbindungen: 0,9 %

In einem Vergleichsversuch wurden 30 g des obengenannten Eisengranulats, ohne vorher zu tempern, 5 Stunden bei 380°C im Wasserstoffstrom reduziert. Der unter gleichen Bedingungen durchgeführte Hydrierversuch erbrachte nur 18,2 % Hexamethylendiamin neben 73,8 % Ausgangsmaterial und 7,9 % Nebenprodukte, die nicht identifiziert wurden.

### Beispiel 2

160,8 g des auch in Beispiel 1 eingesetzten Eisengranulats wurde mit einer Lösung aus 20 g H₂O und 2,01 g (NH₄)₂Cr₂O₇ getränkt und bei 100°C getrocknet. Anschließend wurde das so behandelte Eisengranulat 22 Stunden im Glühofen bei einer Ofentemperatur von 600°C unter Luftzutritt getempert (Gewichtszunahme: 30 %).

28,7 g des so gewonnenen Materials wurden 121 Stunden bei 400°C in einem Wasserstoffstrom, der 3 Vol.-% NH₃ enthielt, reduziert. Mit dem so erhaltenen Katalysator wurden 40 g Adiponitril in Gegenwart von 40 g Ammoniak in einem 0,25 1-Schüttelautoklaven hydriert. Dabei wurde der Autoklav 3,7 Stunden auf 120°C und 250 bis 300 bar gehalten. Das entstandene Reaktionsprodukt wies folgende Zusammensetzung auf:
- Hexamethylendiamin: 98,9 %
- Bishexamethylentriamin: 0,2 %
- Azacycloheptan: 0,5 %
- 1,2-Diaminocyclohexan: 0,2 %
- unbekannte Nebenprodukte: 0,2 %

### Beispiel 3

180 g des auch in Beispiel 1 eingesetzten Eisengranulats wurden mit einer Lösung versetzt, die aus 1,93 g Ru(NO₃)₃ und 100 g Wasser hergestellt wurde, dann wurde im Rotationsverdampfer eingedampft. Anschließend wurde das getrocknete Eisengranulat 22 Stunden im Glühofen bei einer Ofentemperatur von 620°C unter Luftzutritt getempert.

41,5 g des anoxidierten Eisens wurde 6 Stunden bei 380°C in einem Wasserstoffstrom reduziert. Der so erhaltene Katalysator wurde unter Ausschluß von Luft in einen 0,25 l-Autoklaven überführt und dieser anschließend mit 40 g Adiponitril sowie mit 40 g NH₃ beschitkt. Die Hydrierung erfolgte bei 120°C und 240 bis 300 bar in 4 Stunden. Das Reaktionsprodukt hatte folgende Zusammensetzung:
- Hexamethylendiamin: 98,5 %
- Bishexamethylentriamin: 0,1 %
- Adiponitril: 0,2 %
- Azacycloheptan: 0,5 %
- 1,2-Diaminocyclohexan: 0,2 %
- andere Nebenprodukte: 0,6 %

### Beispiel 4

Für eine kontinuierliche Hydrierung von Adiponitril wurden 15 ml (32 g) des in Beispiel 1 hergestellten anoxidierten Eisengranulats in ein senkrecht angeordnetes Druckrohr (Durchmesser 14 mm, Länge 70 cm) gebracht. Durch das Druckrohr wurde von oben nach unten unter einem Druck von 280 bar Wasserstoff geleitet, wobei die Temperatur im Rohr auf 300°C gehalten wurde. Nach einer Reduktionszeit von 22 Stunden war die Herstellung des gebrauchsfertigen Katalysators beendet, so daß mit der Hydrierung von Adiponitril begonnen werden konnte.

Hierzu wurde ein Gemisch von Adiponitril und Ammoniak im Gewichtsverhältnis 1:1,5 kontinuierlich von oben auf die Katalysatorschicht geleitet, während bei einem ständigen, ebenfalls von oben nach unten geleiteten Zustrom von Wasserstoff der Druck in der Hydrierapparatur auf 280 bar gehalten wurde. Die Flüssigkeit rieselte über den Katalysator nach unten in einen Druckabscheider. Am Kopf des Abscheiders wurde Wasserstoff entspannt, um im Reaktionsrohr einen kontinuierlichen Gasstrom zu erzeugen. Bei einer Hydriertemperatur von 118°C und einer Katalysatorbelastung von 0,53 g Adiponitril/ml Katalysator x h und einer Abgasmenge von 50 l/h ergab sich nach 2016 Stunden folgende Produktzusammensetzung:
- Hexamethylendiamin: 98,8 %
- Bishexamethylentriamin: 0,6 %
- Azacycloheptan: 0,2 %
- 1,2-Diaminocyclohexan: 0,2 %
- unbekannte Nebenprodukte: 0,2 %

Der Katalysator zeigte nach 3108 Betriebsstunden noch keinen Aktivitätsabfall.

### Beispiel 5

160 g des auch in Beispiel 1 eingesetzten Eisengranulats wurde mit einer Lösung getränkt, die aus 4,0 g RuCl₃, 0,31 g JrCl₄ · H₂O und 18 g H₂O hergestellt wurde. Das getränkte Eisengranulat wurde bei 110°C getrocknet und anschließend 22 Stunden bei 600°C unter Luftzutritt getempert (Gewichtszunahme: 28,8 %).

30 g des so anoxidierten Eisens wurden 8 Stunden im Wasserstoffstrom (30 l H₂/h) bei 400°C reduziert. Der so hergestellte Katalysator wurde im Wasserstoffstrom abgekühlt und unter Luftausschluß in einen 0,25 l Schüttelautoklaven gegeben; 40 g Adiponitril und 40 g flüssiges Ammoniak wurden in den Autoklaven gegeben und die Hydrierung bei 120°C und 220 bis 300 bar durchgeführt.

Die Hydrierung wurde nach 190 Minuten beendet. Das Reaktionsprodukt wurde gaschromatographisch analysiert und zeigte folgende Zusammensetzung:
- Hexamethylendiamin: 98,2 %
- Bishexamethylentriamin: 0,3 %
- Azacycloheptan: 0,7 %
- 1,2-Diaminocyclohexan: 0,2 %
- unbekannte Verbindungen: 0,6 %

## Patentansprüche

1. Verfahren zur Herstellung eines geformten Eisenkatalysators zur Hydrierung von Nitrilen, dadurch gekennzeichnet, daß man Eisenschwamm, der mindestens zu 85% aus Eisen besteht und bei der Direktreduktion von Eisenerz unterhalb der Schmelztemperatur anfällt, in Form von Granulat oder einer anderen stückigen Form mittels gasförmigen Sauerstoff bei 200 bis 800° C bis zu einer Gewichtszunahme von mindestens 5% und maximal 32% anoxidiert und anschließend im Wasserstoffstrom, der gegebenenfalls untergeordnete Mengen an nicht oxidierenden Gasen enthalten kann, bei 200 - 500° C reduziert.

2. Verfahren gemäß Anspruch 1, dadurch gekennzeichnet, daß man die Oxidation unter Verwendung von Luft bis zu einer Gewichtszunahme von max. 32% durchführt.

3. Verfahren gemäß Anspruch 1 und 2, dadurch gekennzeichnet, daß man den Eisenschwamm vor oder nach dem Oxidationsschritt, jedoch vor der Hydrierung, mit einem als Aktivator wirkenden Metall dotiert.

4. Verfahren zur Herstellung von primären Aminen durch katalytische Hydrierung der entsprechenden Nitrile, dadurch gekennzeichnet, daß man als Hydrierkatalysatoren gemäß Anspruch 1 bis 3 erhältliche Eisenkatalysatoren verwendet.

5. Verfahren gemäß Anspruch 4, dadurch gekennzeichnet, daß man den anoxidierten Eisenschwamm unmittelbar in dem Reaktionsraum, in dem die Hydrierung des Nitrils stattfinden soll, bei 200 bis 400° C und einem Wasserstoffdruck von 10 bis 400 bar reduziert und anschließend die Hydrierung des Nitrils in Gegenwart von Ammoniak bei 80 bis 180° C und einem Druck von 20 bis 400 bar durchführt.

6. Verfahren gemäß Anspruch 4 und 5, dadurch gekennzeichnet, daß man als Nitril Adipinsäuredinitril verwendet.

## Claims

1. A process for the production of a shaped iron catalyst for the hydrogenation of nitriles, characterized in that iron sponge obtained in the direct reduction of iron ore below the melting temperature is partly oxidized with gaseous oxygen at 200 to 800°C in the form of granules or other particles and is then reduced in a stream of hydrogen optionally containing small quantities of non-oxidizing gases at 200 to 500°C.

2. A process as claimed in claim 1, characterized in that oxidation is carried out with air to an increase in weight of at most 32%.

3. A process as claimed in claims 1 and 2, characterized in that the iron sponge is doped with a metal acting as activator before or after the oxidation step, but before the hydrogenation step.

4. A process for the production of primary amines by catalytic hydrogenation of the corresponding nitriles, characterized in that iron catalysts obtainable by the process claimed in claims 1 to 3 are used as the hydrogenation catalysts.

5. A process as claimed in claim 4, characterized in that the partly oxidized iron sponge is reduced directly in the reaction zone in which the nitrile is to be hydrogenated at 200 to 400°C and under a hydrogen pressure of 10 to 400 bar and the nitrile is then hydrogenated in the presence of ammonia at 80 to 180°C and under a pressure of 20 to 400 bar.

6. A process as claimed in claims 4 and 5, characterized in that adipic acid nitrile is used as the nitrile.

## Revendications

1. Procédé pour la fabrication d'un catalyseur au fer formé pour l'hydrogénation de nitriles, caractérisé en ce que l'on oxyde anodiquement du fer spongieux, composé d'au moins 85% de fer, obtenu par la réduction directe de minerai de fer à une température inférieure à la température de fusion, sous forme de produit granulé ou sous une autre forme fragmentée,au moyen d'oxygène gazeux à une température de 200 à 800°C jusqu'à l'obtention d'une augmentation de poids de minimum 5% et maximum 32%, et que l'on réduit ensuite à une température de 200 à 500°C dans un courant d'hydrogène pouvant éventuellement contenir des quantités secondaires de gaz non oxydants.

2. Procédé selon la revendication 1, caractérisé en ce que l'oxydation a lieu avec de l'air jusqu'à une augmentation de poids de maximum 32%.

3. Procédé selon les revendications 1 et 2, caractérisé en ce qu'on dope le fer spongieux avant ou après l'étape d'oxydation, mais avant l'étape d'hydrogénation, avec un métal agissant comme activant.

4. Procédé pour la fabrication d'amines primaires par hydrogénation catalytique des nitriles correspondants, caractérisé en ce que l'on utilise comme catalyseur d'hydrogénation un catalyseur au fer pouvant être obtenu selon les revendications 1 à 3.

5. Procédé selon la revendication 4, caractérisé en ce qu'on réduit le fer spongieux oxydé anodiquement directement dans l'espace réactionnel où l'hydrogénation du nitrile doit avoir lieu,à une température de 200 à 400 °C et avec une pression d'hydrogène de 10 à 400 bar, et qu'on effectue ensuite l'hydrogénation du nitrile en présence d'ammoniac à une température de 80 à 180°C et sous une pression de 20 à 400 bar.

6. Procédé selon les revendications 4 et 5, caractérisé en ce qu'on utilise comme nitrile de l'adiponitrile.
